# EUROPEAN PATENT APPLICATION

(11) **EP 3 696 279 A1**
(43) Date of publication of application: **19.08.2020**
(21) Application number: 19156991.2
(22) Date of filing: 13.02.2019
(51) Int. Cl.: C12Q 1/6809

(54) **METHODS FOR NONINVASIVE PRENATAL TESTING OF FETAL ABNORMALITIES**

(71) Applicant: NIPD GENETICS PUBLIC COMPANY LIMITED, Nicosia 2409 (CY)
(72) Inventor: Koumbaris, George, 2565 Lythrodontas (CY); Ioannides,Marios, Egkomi 2416 Nicosia (CY); Patsalis ,Philippos C., Egkomi , Nicosia 2402 (CY); Achilleos, Archilleas, 3313 Limassol (CY); Tsangaras, Kyriakos, 4040 Limassol (CY); Loizides, Charalambos, Strovolos, Nicosia 2013 (CY)
(74) Representative: CH Kilger Anwaltspartnerschaft mbB

(57) **Abstract**

The present invention relates to a method for the detection of genetic and or genomic abnormalities in a mixed sample, comprising the steps of biochemical and *in-silico* enrichment of a subset of cell-free DNA fragments derived from the mixed sample. The invention utilizes a pool of long DNA probes to enrich for sequences of interest in the mixed sample, followed by massive parallel sequencing and a computer-based analysis of the enriched sub-population to detect a risk of genetic and or genomic abnormalities in the said sub-population of the mixed sample. The computer-based part of the method does not necessarily require alignment on a reference genome nor calibration values using reference samples. The method also comprises a kit for performing the invention.

## Description

### FIELD OF THE INVENTION

The invention is in the field of biology, medicine and chemistry, in particular in the field of molecular biology and more in particular in the field of molecular diagnostics.

### BACKGROUND OF THE INVENTION

The discovery of cell-free fetal DNA (cffDNA) in maternal plasma has greatly promoted the development of non-invasive prenatal tests. However, most of the developed tests for fetal aneuploidy and micro-deletion detection rely on single normalized values derived from read-depth information. Although these tests can be considered as a significant improvement over current methods, their clinical sensitivities do not exceed more than 99%. Especially when the proportion of cffDNA in the maternal circulation is below 4% and even with next generation sequencing (NGS) technology which has a high sensitivity, obtaining sufficient accuracy for non-invasive prenatal testing (NIPT) is challenging.

### SUMMARY OF THE INVENTION

The present invention provides a method of double enrichment for placenta derived cell-free DNA (cfDNA) fragments in a mixed biological sample comprising fetal and maternal cell-free DNA, using biochemical and *in silico* approaches that increase the signal-to-noise ratio through the use of long capture-probes, fragment-size analysis, utilization of hot spots of non-random fragmentation (HSNRF) and a novel bioinformatics framework. The method enables high-sensitivity non-invasive detection of fetal abnormalities by utilizing the information arising from the enrichment of fetal cell-free DNA fragments in a prenatal sample using long DNA probes that capture cell free DNA fragments and/or enrich for hot spots of non-random fragmentation.

The invention also includes a novel computer-based method that organizes observed data (sequencing reads) into meaningful structures that improve the signal-to-noise ratio in cell-free DNA analysis of samples comprising a mixture of cell-free DNA (cfDNA) fragments. The invented method groups a plurality of DNA sequences in a way that the degree of homology between these DNA sequences is maximal if they truly originate from the same structure and minimal otherwise. Specific sequence patterns are identified in said data and used to allocate them in predetermined regions of interest. The said method can be applied on discovering particular structures in DNA sequence data without the need of any prior knowledge, such as alignment on a human reference genome and/or calibration values using reference samples and is herein applied for the non-invasive prenatal detection of fetal chromosomal abnormalities, such as aneuploidies, microdeletions, microduplications and point mutations.

As such, in a first aspect the invention relates to a method of detecting fetal genetic/genomic abnormalities in a mixed sample comprising maternal and fetal cfDNA, the method comprising the steps of:
(i) obtaining a mixture of cfDNA from an individual;
(ii) preparing a sequencing library from the cfDNA;
(iii) enriching the cfDNA library using long DNA probes;
(iv) sequencing the enriched cfDNA library;
(v) performing statistical analysis to determine a risk of chromosomal and/or other genetic abnormality in the fetal DNA.
wherein the risk of chromosomal and/or other genetic abnormality in the fetal DNA is classified based on a double enrichment method, comprising genomic hybridization and a computer-based method, that enhances the signal-to-noise ratio in said analysis.

The method wherein enriched fragments are subject to a procedure comprising the steps of:
(i) grouping sequenced reads, that can be paired, based on nucleotide patterns, that is, but not limited to, the extent of overlap in terms of nucleotide composition;
(ii) grouping and/or annotating at least a subset of the plurality of sequenced reads based on nucleotide patterns, that is, but not limited to, the sequence similarity of the, at least 20, outermost nucleotides;
(iii) matching predetermined nucleotide sequences to the reads in (i) and (ii);
(iv) removing duplicate sequenced reads;
(vi) utilizing information obtained from (i) to (iv) to perform statistical analysis to determine a risk of chromosomal and/or other genetic abnormality in the fetal DNA.

In another aspect, the present invention provides a kit for carrying out the said invention. In one embodiment the kit comprises:
a. probes that hybridize to at least one location in the nucleic acid fragments, wherein said at least one location partially or completely encompasses the nucleic acid fragment where a hot spot for non-random fragmentation (HSNRF) lies and, optionally,
b. reagents and/or software for carrying out the invention and detecting genetic and/or genomic abnormalities in a sample.

### BRIEF DESCRIPTION OF THE FIGURES

Figure 1 shows a proof-of-principle experiment wherein 92 diploid samples and four trisomy 21 samples were classified using said method.
Figure 2 shows a proof-of-principle experiment wherein 91 normal samples and two 22q11.2 deletion syndrome cases were classified for microdeletion (22q11.2 deletion syndrome) using said method.
Figure 3 shows a Kernel density estimate of fragment-size distribution from a representative sample using (A) all fragments and (B) only the method-selected fragments.

### DETAILED DESCRIPTION OF THE INVENTION

As such, the invention relates to a method of detecting fetal genetic/genomic abnormalities in a mixed sample comprising maternal and fetal cfDNA, the method comprising the steps of:
(i) obtaining a mixture of cfDNA from an individual;
(ii) preparing a sequencing library from the cfDNA;
(iii) enriching the cfDNA library using long DNA probes;
(iv) sequencing the enriched cfDNA library;
(v) performing statistical analysis to determine a risk of chromosomal and/or other genetic abnormality in the fetal DNA.

Herein, the mixture of nucleic acid fragments is preferably isolated from a sample taken from a eukaryotic organism, preferably a primate, more preferably a human. The sample can be from various tissue types. As such, a sample comprises intrinsically of a mixture of nucleic acid fragments.

In the context of the present invention, the expression "nucleic acid fragments" and "fragmented nucleic acids" can be used interchangeably.

In a preferred embodiment of the method according to the invention, the nucleic acid fragments are circulating cell-free DNA or RNA.

In one embodiment, the sample is a maternal plasma sample comprising cell-free maternal DNA and cell-free fetal DNA (cffDNA).

The invention can also be used with a variety of biological samples. Essentially any biological sample containing genetic material, e.g. RNA or DNA, and in particular cell-free DNA (cfDNA), can be used as a sample in the invention. In one embodiment, the DNA sample originates from a plasma sample containing cell-free DNA (cfDNA). In particular for prenatal testing, the DNA sample contains fetal DNA (e.g., cell-free fetal DNA). In one embodiment for NIPT, the sample is a mixed sample that contains both maternal DNA and fetal DNA (e.g., cell-free fetal DNA (cffDNA)), such as a maternal plasma sample obtained from maternal peripheral blood. Typically for mixed maternal/fetal DNA samples, the sample is a maternal plasma sample, although other tissue sources that contain both maternal and fetal DNA can be used. As used herein, the term "mixed sample" refers to a mixture of at least two biological samples originating from different sources, e.g., maternal/fetal DNA samples.

Depending upon the circumstances, the biological sample encompasses: embryonic DNA and maternal DNA, tumor-derived DNA and non-tumor derived DNA, pathogen DNA or host DNA and DNA derived from a transplanted organ and DNA derived from the host.

Therefore, in the context of non-invasive diagnosis, the sample is a mixed sample, wherein said mixed sample is selected from the group comprising (i) embryonic DNA and maternal DNA, (ii) tumor derived DNA and non-tumor derived DNA, (iii) pathogen DNA and host DNA and (iv) DNA derived from a transplanted organ and DNA derived from the host.

Maternal plasma can be obtained from a peripheral whole blood sample from a pregnant subject and the plasma can be obtained by standard methods. As little as 1-4 ml of plasma is sufficient to provide suitable DNA material for analysis according to the method of the disclosure. Total cell-free DNA can then be extracted from the sample using standard techniques, non-limiting examples of which include a QIAsymphony protocol (QIAGEN) suitable for cell-free fetal DNA isolation or any other manual or automated extraction method suitable for cell-free DNA isolation.

In the context of the present invention, the term "subject" refers to animals, preferably mammals, and, more preferably, humans. The "subject" referred to herein is a pregnant subject, and, therefore, preferably, a female subject. The pregnant subject may be at any stage of gestation. The "subject" may get pregnant naturally or by means of artificial techniques. As used herein, the term "subject" also refers to a subject suffering from or suspected of having a tumor. Said subject can be subjected to organ transplantation or experienced a pathogen infection after a transplant or independently from a transplant.

For the biological sample preparation, typically, DNA is extracted using standard techniques known in the art, a non-limiting example of which is the QIAsymphony (QIAGEN) protocol.

Following isolation, the cell-free DNA of the sample is used for sequencing library construction to make the sample compatible with a downstream sequencing technology, such as Next Generation Sequencing. Typically, this involves ligation of adapters onto the ends of the cell-free DNA fragments, followed by amplification. Sequencing library preparation kits are commercially available or can be developed.

Preferably, in the method according to the invention, the first and second nucleic acid fragments are selected from the groups comprising:
i. embryonic DNA and maternal DNA,
ii. tumor derived DNA and non-tumor derived DNA,
iii. pathogen DNA and host DNA,
iv. DNA derived from a transplanted organ and DNA derived from the host.

In the context of the present invention, the terms fetus and embryo are used interchangeably.

In one embodiment, preferably the probes span a hotspot of non-random fragmentation (HSNRF) site such that only the 5' end of the fragmented nucleic acid is captured by the probe.

In another embodiment, the probes span a HSNRF site such that only the 3' end of the cell-free nucleic acids arising from HSNRF can bind to the probe.

In another preferred embodiment, the probes span both HSNRF sites associated with a fragmented nucleic acid such that both the 5' and the 3' end of a cell-free nucleic acid associated with the given HSNRF site are captured by the probe.

In another embodiment, mixtures of the above are used.

Ideally, in the method according to the invention, the probes used for enrichment of cfDNA are double-stranded DNA fragments and, (i) each probes is between 100-500 base pairs in length, (ii) each probe has a 5' end and a 3' end, (iii) preferably each probe binds to the HSNRF at least 10 base pairs away, on both the 5' end and the 3' end, from regions harboring copy number variations (CNVs), segmental duplications or repetitive DNA elements, and (iv) the GC content of each probes is between 19% and 80%.

In general, the hybridization step, preferably with probes, can be carried out before the sequencing library is created or after the library has been created.

The region(s) of interest on the chromosome(s) of interest where the HSNRF lie are enriched by hybridizing the pool of probes to the sequencing library, followed by isolation of those sequences within the sequencing library that bind to the probes. In one embodiment, the probe spans a HSNRF site such that only the 5' end of the fragmented cell-free nucleic acids is captured by the probe. In another embodiment the probe spans a HSNRF site such that only the 3' end of the fragmented cell-free nucleic acids arising from HSNRF can bind to the probe. In another preferred embodiment, the probe spans both HSNRF sites associated with a fragmented nucleic acid such that both the 5' and the 3' end of a cell-free nucleic acid associated with the given HSNRF site are captured by the probe.

To facilitate isolation of the desired enriched sequences (HSNRF), typically the probe sequences are modified in such a way that sequences that hybridize to the probes can be separated from sequences that do not hybridize to the probes. Typically, this is achieved by fixing the probes to a support. This allows for physical separation of those sequences that bind the probes from those sequences that do not bind the probes. In one embodiment, each sequence within the pool of probes can be labeled with biotin and the pool can then be coupled to beads coated with a biotin-binding substance, such as streptavidin or avidin. In a preferred embodiment, the probes are labeled with biotin and bound to streptavidin-coated magnetic beads, thereby allowing separation by exploiting the magnetic property of the beads. The ordinarily skilled artisan will appreciate, however, that other affinity binding systems are known in the art and can be used instead of biotin-streptavidin/avidin. For example, an antibody-based system can be used in which the probes are labeled with an antigen and then bound to antibody-coated beads. Moreover, the probes can incorporate on one end a sequence tag and can be bound to a support via a complementary sequence on the support that hybridizes to the sequence tag. Furthermore, in addition to magnetic beads, other types of supports can be used, such as polymer beads and the like.

In certain embodiments, the members of the sequencing library that bind to the pool of probes are fully complementary to the probes. In other embodiments, the members of the sequencing library that bind to the pool of probes are partially complementary to the probes. For example, in certain circumstances it may be desirable to utilize and analyze data that are from DNA fragments that are products of the enrichment process but that do not necessarily belong to the genomic regions of interest (i.e. such DNA fragments could bind to the probes because of part homologies (partial complementarity) with the probes and when sequenced would produce very low coverage throughout the genome in non-probes coordinates).

Following enrichment of the sequence(s) of interest using the probes, thereby forming an enriched library of DNAs with HSNRF sites, the members of the enriched HSNRF library are eluted from the solid support, are amplified, and sequenced using standard methods known in the art.

Next Generation Sequencing is typically used, although other sequencing technologies can also be employed, which provide very accurate counting in addition to sequence information. Accordingly, other accurate counting methods, such as digital PCR, single molecule sequencing, nanopore sequencing, and microarrays can also be used instead of NGS.

The invention relates to a method wherein the nucleic acid fragment to be detected, is present in the mixture at a concentration lower than a nucleic acid fragment from the same genetic locus but of different origin. That means that if a particular locus is selected, e.g. the maternal copy will be present 100 times and the copy from the fetus only once, in the solution comprising the isolated cfDNA. In the case of fetal derived cfDNA, the fetal derived component of a mixed sample can have a range of possible values. For example, the range of fetal material in a mixed sample can be in the range of 2% - 30%. Frequently, fetal derived fragments are around 10% of the total DNA of a mixed sample. More importantly, in some compositions of mixed samples the fetal DNA component of the sample can be less than 5%. Particularly, in some sample compositions the fetal derived material is 3%, or less, of the total sample.

The present method is particularly suited to analyze such low concentrations of target cfDNA. In the method according to the invention, the nucleic acid fragment to be detected or the origin of which is to be determined and the nucleic acid fragment from the same genetic locus but of different origin are present in the mixture at a ratio selected from the group of 1:2, 1:4, 1:10, 1:20, 1:50, 1:100, 1:200, 1:500, 1:1000, 1:2000 and 1:5000. The ratios are to be understood as approximate ratios, which means plus/minus 30%, 20% or 10%. A person skilled in the art knows that such ratios will not occur at exactly the numerical values cited above. The ratios refer to the number of locus specific molecules for the rare type to the number of locus molecules for the abundant type.

In one embodiment, the probes are provided in a form that allows them to be bound to a support, such as biotinylated probes. In another embodiment, the probes are provided together with a support, such as biotinylated probes provided together with streptavidin-coated magnetic beads.

In a particular embodiment, the GC content of the probes or probes is between 10% and 70%, preferably 15% and 60%, more preferably 20% and 50%.

The described method wherein the result could be combined with further statistical tests from a group comprising a t-test, a bivariate nonparametric bootstrap test, a stratified permutation test and a fragment sizes proportion test

In the context of the present invention, the term "partially" refers to a region (location) of 10, 20, 30 or 40 bases of the nucleic acid fragment from the 5'-end or from the 3'-end. Consequently, as used herein, the term "completely" refers to a region (location), which encompasses 100% of the nucleic acid fragment. According to the method of the present invention, the probes hybridize to at least one location within the nucleic acid fragment. However, more than one location within the same nucleic acid fragment can be also targeted by the probes.

In one embodiment, the kit comprises a container consisting of the pool of probes, software, and instructions for performing the method.

In addition to the pool of probes, the kit can comprise one or more of the following (i) one or more components for isolating cell-free DNA from a biological sample, (ii) one or more components for preparing and enriching the sequencing library (e.g., primers, adapters, buffers, linkers, DNA modifying enzymes, ligation enzymes, probes, polymerase enzymes and the like), (iii) one or more components for amplifying and/or sequencing the enriched library, and/or (iv) software for performing statistical analysis.

Accordingly, in another embodiment of the invention, it is preferred that the fragment to be detected or determined is of smaller size than the second fragment. Depending on the tissue of origin, different fragment sizes are expected across HSNRF locations. Since cells of each tissue are differentiated to perform specific functions, then different patterns of genetic activity are expected to be seen by each tissue type. Since, gene activation is dependent on the tertiary structure of DNA then different tissues will have slightly different conformations which lead to different cut sites and consequently different fragment sizes. For example, it has been demonstrated that cell-free DNA of placental origin is likely to be of smaller size when compared to cell-free DNA of maternal origin.

### EXAMPLES

The present invention is further illustrated by the following examples, which should not be construed as further limiting.

### Example 1: Sample collection and library preparation

The general methodology for the double enrichment of placenta derived DNA fragments in a mixed biological sample comprising fetal and maternal cell-free DNA for non-invasive prenatal diagnosis purposes is explained. In this example, methods for collecting and processing a maternal plasma sample (containing maternal and fetal DNA) are described. The same approach can be followed in other medically useful cases, such as, but not limited to oncology, genetic mutation, transplantation, and assessment of pathogen load.

### Sample collection

Plasma samples were obtained anonymously from pregnant women after the 10^{th} week of gestation. Protocols used for collecting samples were approved by the appropriate Bioethics Committee, and informed consent was obtained from all participants.

### Sample extraction

Cell-free DNA was extracted from 1-4 ml plasma from each individual using a manual or automated extraction method suitable for cell-free DNA isolation such as for example, but not limited to, QIAsymphony protocol suitable for cell-free DNA isolation (QIAGEN).

### Sequencing library preparation

Extracted cell-free DNA from maternal plasma samples was used for sequencing library construction. Initially, 5' and 3' overhangs were repaired while 5' ends were phosphorylated. Reaction products were purified using AMPure XP beads (Beckman Coulter). Subsequently, sequencing adaptors were ligated to both ends of the DNA, followed by purification using AMPure XP beads (Beckman Coulter). Nicks were removed in a fill-in reaction with a polymerase and were subsequently purified using AMPure XP beads (Beckman Coulter). Library amplification was performed using another polymerase enzyme (Koumbaris et al. (2016) Clinical chemistry 62(6):848-855). The final library products were purified using AMPure XP beads (Beckman Coulter) and measured by spectrophotometry.

### Example 2: Probe Design and Preparation

This example describes preparation of custom probes for the detection of HSNRF. The genomic target-loci used for probes design were selected based on their GC content and their distance from repetitive elements (minimum 50 bp away). Probes size can be variable. In one embodiment of the method the probes range from 100-500 bp in size and are generated through a PCR-based approach. The probes were prepared by simplex polymerase chain reaction using standard Taq polymerase, primers designed to amplify the target-loci, and normal DNA used as template. In a preferred embodiment, the probe spans a HSNRF site such that only the 5' end of the fragmented nucleic acid is captured by the probe. In another embodiment, the probe spans a HSNRF site such that only the 3' end of the cell-free nucleic acids arising from HSNRF can bind to the probe. In another preferred embodiment, the probe spans both HSNRF sites associated with a fragmented nucleic acid such that both the 5' and the 3' end of a cell-free nucleic acid associated with the given HSNRF site are captured by the probe.

### Example 3: probe hybridization and amplification

This example describes the method of target capture of nucleic acids by hybridization using probes, said probes preferably spanning HSNRF, followed by quantitation of captured sequences by Next Generation Sequencing (NGS).

### Probe biotinylation

Probes were prepared for hybridization, starting with blunt ending followed by purification. They were then ligated with a biotin adaptor and purified. Probes were denatured prior to immobilization on streptavidin coated magnetic beads.

### Probe hybridization

Amplified libraries were mixed with blocking oligos, Cot-1 DNA, Salmon Sperm DNA, hybridization buffer, blocking agent, and denatured. Denaturation was followed by 30 minutes incubation at 37°C. The resulting mixture was then added to the biotinylated probes and incubated for 12-48 hours 60-70C. After incubation, the enriched libraries were washed as described previously and DNA was eluted by heating. Eluted products were amplified using outer-bound adaptor primers. Enriched amplified products were pooled equimolarly and sequenced on a suitable platform.

### Example 4: Bioinformatics Analysis

The inventors have developed a computer-based method that organizes observed data (sequencing reads) into structures that allow the selection of subsets of the sequenced reads from an enriched sample in order to increase the signal to noise ratio in a sample comprising a mixture of cell-free DNA (cfDNA) fragments. The invented method groups and/or annotates a plurality of DNA sequences in a way that the degree of homology between these DNA sequences is maximal if they truly originate from the same group and minimal otherwise. Specific sequence patterns are then identified in said data and used to allocate them in predetermined regions of interest. In the current invention the method is used for the non-invasive detection of fetal chromosomal abnormalities, such as aneuploidies, microdeletions, and microduplications in a sample comprising a mixture of cell-free fetal DNA (cffDNA) and maternal DNA.

In a preferred embodiment, each sample's FASTQ files are not aligned against a version of the human reference genome. The fragment size information and/or class is obtained using or the length of single reads (when greater than 150bp) or overlapping sequence information of pair end reads. Identical reads are removed and either de-novo assembly or matching with pre-determined targets of short sequences was performed.

The ordinarily skilled artisan will appreciate the presence of many freely available and well-established tools to perform matching of sequences.

The algorithm is a collection of data processing, mathematical and statistical model routines arranged as a series of steps. The algorithm's steps aim in deciding the relative copy number state of a chromosome of interest and/or subchromosomal region of interest in the enriched sample and is used for the detection of whole or partial chromosomal abnormalities, such as aneuploidies of chromosomes 13, 18, 21, X, Y or any other chromosome, as well as microdeletion/microduplication syndromes and other small size mutations.

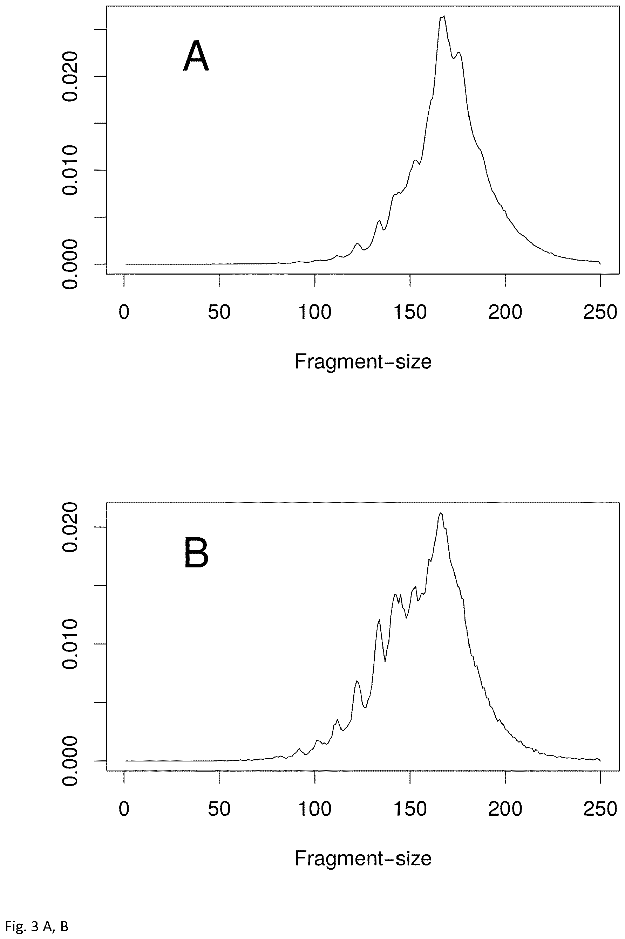

A key characteristic of the method is the double enrichment of a sample with respect to fetal (placental) fraction by selecting a subset of enriched fragments and thus resulting in an increase of the signal to noise ratio (fetal fraction to maternal fraction, i.e. proportion of placental derived fragments). To this end, the developed method identifies and utilizes fragments with high likelihood of originating from the placenta. Figure 3 shows the estimated size distribution of the method-selected fragments as opposed to the size distribution of all fragments of an indicative sample, illustrating the enrichment of shorter fragments, which primarily originate from fetal (placental) tissues.

In one embodiment of the method, the enriched sample is sequenced using a 2x75bp sequencing method. In said embodiment, sequenced fragments are grouped according to size, based on the extend of overlap or absence of overlap of their paired-end reads. Each fragment is classified accordingly into two groups, short (group 1) and long (group 2). Then HSNRF are identified by utilizing the sequence similarity of the, at least 20, outermost nucleotides of fragments in group 1. Fragments from group 2, whose ends overlap with identified HSNRF are then used to construct group 3. Then all fragments from group 1, and all fragment from group 3 are retained for subsequent analysis. Said cfDNA fragments of group 1 are shorter in size and are enriched for cell-free fetal DNA. Said cfDNA fragments of group 3 are longer in size, but are also enriched for cell-free fetal DNA because their ends overlap with HSNRF. cfDNA fragments of group 2 whose ends do not overlap with HSNRF have higher likelihood of being derived from the mother and are discarded from all subsequent analyses. Figure 3 shows the size distribution of the method-selected fragments (group 1 and group 3) compared to the size distribution of all fragments in a typical sample, illustrating the enrichment of fetal (placental) cfDNA fragments.

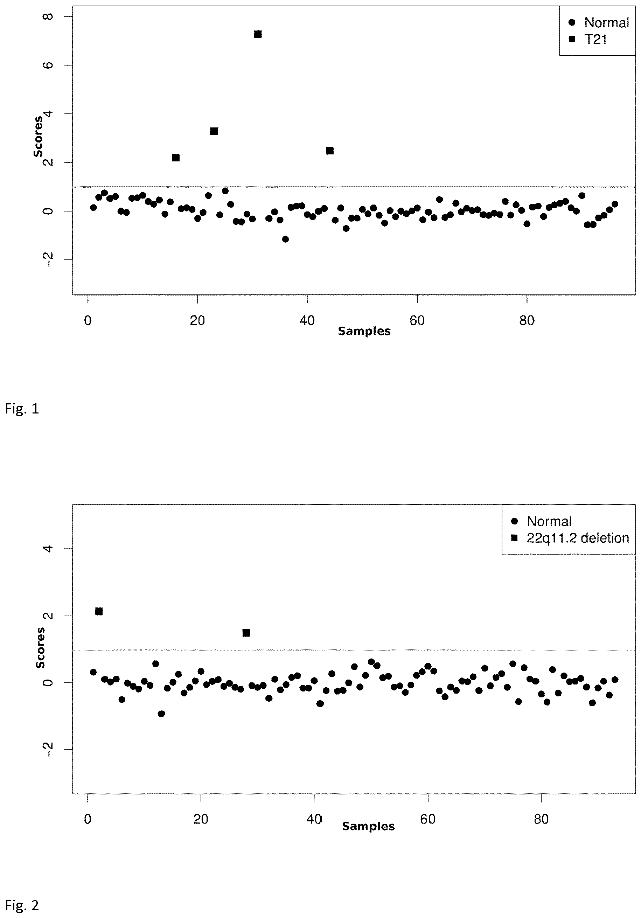

Following mapping of all fragments from group 1 and group 3 to probe sequences, a classification score is computed for each tested sample by comparing the number of retained fragments on a target chromosome, e.g., chromosome 21 (Figure 1) and/or a target subchromosomal region, e.g., 22q11.2 (Figure 2) with the number of fragments on reference chromosome/s and/or subchromosomal region/s. In another embodiment of the method, said steps can be performed following alignment of sequenced reads to a reference genome

## Claims

1. A method for the detection of a chromosomal abnormality in a mixed sample, comprising the steps of:
(a) obtaining a biological sample, the sample comprising a mixture of cell-free DNA (cfDNA) fragments,
(b) preparing a sequencing library from the cfDNA fragments,
(c) hybridizing one or more probes to at least one or more cfDNA fragments,
(d) isolating cfDNA fragments of the library that bind to the probes,
(e) sequencing the cfDNA fragments of the library that bind to the probes,
(f) utilizing the size, start and/or stop information of each or a subset of the enriched cfDNA fragments from steps (c-e) to select a fraction of cfDNA fragments hybridized to said one or more probes
wherein step (f) is associated with the computation of several statistical tests.

2. The method according to claim 1, wherein the selection step (f) comprises the steps of:
(i) categorizing cfDNA fragments into a first and a second cluster distribution,
(ii) detecting hotspots of non-random fragmentation (HSNRF) using cfDNA fragments of the first cluster distribution,
(iii) categorizing cfDNA fragments of the second cluster distribution into a third cluster,
(iv) combining the cfDNA fragments of the first cluster distribution with cfDNA fragments of the third cluster distribution

3. The method according to claim 2, wherein the first cluster distribution comprises cfDNA fragments having a length less than or equal to 120bp or 125bp or 130bp or 135bp or 140bp or 145bp or 150bp or 155bp and the second cluster distribution comprises cfDNA fragments having a length higher than 120bp or 125bp or 130bp or 135bp or 140bp or 145bp or 150bp or 155bp, respectively.

4. The method according to any of the claims 2 to 3, wherein the third cluster comprises selecting cfDNA fragments from second cluster whose ends overlap with HSNRF detected from (2)(ii).

5. The method according to any of the claims 1 to 4, wherein the probes are long DNA molecules and:
(i) each probe is between 100-500 base pairs in length,
(ii) each denatured probe has a 5'-end and a 3'-end,
(iii) preferably, each probe binds to a HSNRF at least 10 base pairs away, on both the 5'-end and the 3'-end, from regions harboring copy number variations (CNVs), segmental duplications or repetitive DNA elements, and
(iv) the GC content of each probe is between 19% and 80%.

6. The method according to any of the claims 1 to 5, wherein the sample is a mixed sample selected from the group comprising: (i) embryonic DNA and maternal DNA, (ii) tumor derived DNA and non-tumor derived DNA, (iii) pathogen DNA and host DNA and (iv) DNA derived from a transplanted organ and DNA derived from the host.

7. The method according to any of the claims 1 to 6, wherein the method further comprises the step of combining statistical tests selected from a group comprising, but not limited to, a t-test, a bivariate nonparametric bootstrap test, a stratified permutation test, a nonparametric test, ANOVA, and a fragment-size proportion test.

8. The method according to any of the claims 1 to 7 for use in diagnosing and/or screening for a genetic abnormality in a sample.

9. The method according to claim 8, wherein the genetic abnormality is selected from the group including, but not limited to:
(a) aneuploidies of chromosomes 13, 18, 21 and/or X, Y.
(b) structural abnormalities, including but not limited to copy number changes including microdeletions and microduplications, insertions, deletions, translocations, and small-size mutations including point mutations.

10. Probes for use in a method according to any of the claims 8 and 9.

11. A method for double enrichment of placenta derived fragments in a mixture of cell-free DNA (cfDNA) comprising the steps of:
(a) obtaining a biological sample, the sample comprising a mixture of cell-free DNA (cfDNA) fragments,
(b) preparing a sequencing library from the cfDNA fragments,
(c) hybridizing the cfDNA library to a plurality of probes, said probes preferably spanning at least one HSNRF,
(d) isolating cfDNA fragments of the library that bind to the probes,
(e) sequencing the cfDNA fragments of the library that bind to the probes,
(f) removing duplicate sequenced reads,
(g) selecting short cfDNA fragments,
(h) detecting HSNRF from short cfDNA fragments,
(i) selecting long cfDNA fragments whose ends overlap with HSNRF,
(j) mapping selected cfDNA fragments from (g) and (i) to probe sequences

12. The method according to claim 11, wherein the short cfDNA fragments have a length less than or equal to 120bp or 125bp or 130bp or 135bp or 140bp or 145bp or 150bp or 155bp and the large cfDNA fragments have a length higher than 120bp or 125bp or 130bp or 135bp or 140bp or 145bp or 150bp or 155bp, respectively.

13. The method according to claim 11, wherein the cfDNA fragments from step (j) are used for fetal aneuploidy detection in a non-invasive prenatal diagnostic test.

14. The method according to claim 11, wherein the cfDNA fragments from step (j) are used for fetal microdeletion/microduplication detection in a non-invasive prenatal diagnostic test.

15. The method according to claim 11, wherein the cfDNA fragments from step (j) are used to detect fetal insertions, deletions, translocations, and small-size mutations including point mutations.

16. Kit for performing a non-invasive test for the use in a method according to claims 1 to 15, comprising:
a. probes that hybridize to at least one location in the nucleic acid fragments, wherein said at least one location preferably spans at least one HSNRF, and, optionally,
b. reagents and/or software for performing the method described according to claims 1 to 15.
